# EUROPEAN PATENT SPECIFICATION

(11) **EP 3 903 838 B1**
(45) Date of publication and mention of the grant of the patent: **16.08.2023**
(21) Application number: 21170862.3
(22) Date of filing: 28.04.2021
(51) Int. Cl.: A61L 9/00, F24F 8/26, A61L 2/22, B64D 11/06, B64D 13/06, B64F 5/30

(54) **GASEOUS DECONTAMINATION OF AIRCRAFT**
GASFÖRMIGE DEKONTAMINATION VON LUFT-FAHRZEUGEN
DÉCONTAMINATION GAZEUSE D'AÉRONEF

(30) Priority: 01.05.2020 US 202063018865 P; 21.05.2020 US 202063028186 P
(43) Date of publication of application: 03.11.2021
(73) Proprietor: Hamilton Sundstrand Corporation, Charlotte, NC 28217-4578 (US)
(72) Inventor: PESS, Mathew, West Hartford, CT 06107 (US); BARTOSZ, Lance R., Granby, MA 01033 (US); D'ORLANDO, Paul M., Simsbury, CT 06070 (US)
(74) Representative: Dehns

(56) References cited:
- EP-A1- 2 777 716
- WO-A1-2007/087709
- US-B2- 9 440 510

## Description

### BACKGROUND

Embodiments of the disclosure relate to the sterilization of an aircraft, and more specifically, to a system and method for killing bacteria and viruses present within an aircraft.

Processes for cleaning and decontaminating aircraft after each flight are typically determined by an airline. Such processes typically include picking up garbage, and wiping down surfaces such as tray tables, arm rests, and seat belt buckles. However, existing processes may not be sufficient to kill bacteria or viruses present within all of the passenger occupied areas of an aircraft. EP 2 777 716 relates to decontamination of compartments. WO 2007/087709 relates to sanitization of an aircraft or a vehicle cabin.

### BRIEF DESCRIPTION

A system is provided according to claim 1.

A cleaning system is provided according to claim 10.

A method of sterilizing a passenger area of a vehicle is provided according to claim 13.

Further embodiments are defined by the dependent claims.

### BRIEF DESCRIPTION OF THE DRAWINGS

The following descriptions should not be considered limiting in any way. With reference to the accompanying drawings, like elements are numbered alike:
FIG. 1 is a schematic diagram of an air management system of an aircraft;
FIG. 2 is a perspective view of an aircraft connected to a support system according to an embodiment;
FIG. 3 is a perspective view of an aircraft connected to a support system according to another embodiment;
FIG. 4 is a perspective view of an aircraft connected to a cleaning system according to an example falling outside the scope of the claims; and
FIG. 5 is a schematic diagram of an air management system of an aircraft including a cleaning system according to an embodiment.

### DETAILED DESCRIPTION

A detailed description of one or more embodiments of the disclosed apparatus and method are presented herein by way of exemplification and not limitation with reference to the Figures.

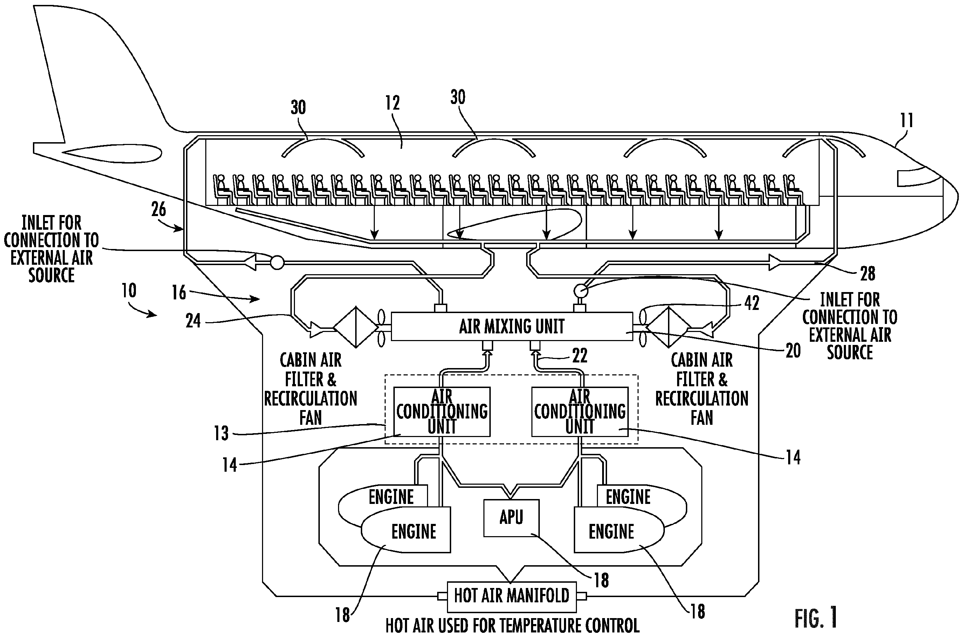

With reference now to FIG. 1, a schematic of an example of an air management system 10 to control the air of a vehicle, such as an aircraft 11 is illustrated. The aircraft 11 includes a pressurized area or cabin 12 that the air management system 10 controls. The cabin 12 may be configured to house people, cargo, and the like therein. The air management system 10 provides conditioned air to, and removes used or contaminated air from, the cabin 12. The air management system 10 includes an environmental control system 13 having at least one air conditioning unit or pack 14, and a cabin air recirculation sub-system 16. While the air management system 10 is illustrated and described herein with reference to an aircraft 11, it should be understood that the systems and techniques discussed herein may be used for a variety of air management systems 10. For example, the cabin 12 may be replaced with any closed volume to be conditioned. As such, systems described herein may be used with ship air management systems, such as submarines and cruise liners for example, personnel carrier air management systems, bus, trolley, train, or subway air management systems, or any other air management system that requires a continual supply of conditioned air.

As shown in FIG. 1, a medium, such as air for example, is provided from one or more sources 18 to the air management system 10. Examples of suitable sources 18 include but are not limited to an engine of the aircraft 11 and an auxiliary power unit of the aircraft 11. The medium output from these sources 18 is provided to the one or more air conditioning units 14 of the environmental control system 13. Within these air conditioning units 14, the medium is conditioned. This conditioning includes altering one or more of a pressure, temperature, humidity, or flow rate of the medium based on an operating condition of the aircraft. The medium output or discharged from the one or more air conditioning units 14 of the environmental control system 13 may be used maintain a target range of pressures, temperatures, and/or humidity levels within the cabin 12.

The medium discharged from the air conditioning units 14 is provided to an air mixing unit or mixing manifold 20 via one or more outlet ducts 22. Similarly, at least one duct 24 of the cabin discharge air system 16 extends from the cabin 12 to the air mixing unit 20 to deliver air exhausted from the cabin 12 to the air mixing unit 20. Within the air mixing unit 20, the cabin recirculating air is mixed with the medium output from the one or more air conditioning units 14 to achieve a mixed medium having one or more desired parameters, such as temperature, pressure, and humidity for example.

In an embodiment, the mixed medium is delivered to the cabin 12 from the air mixing unit 20 via an air distribution system 26 including one or more conduits 28. As shown, the mixed medium may be delivered to the cabin 12 and cockpit via a ventilation system arranged near a ceiling of the cabin 12. In some embodiments, the mixed medium typically circulates from the top of the cabin 12 toward the floor, and is distributed to a plurality of individual vents 30 of the ventilation system spaced laterally between the front and rear of the cabin 12. It should be understood that the air management system 10 illustrated and described herein is intended as an example only, and that any suitable air management system is within the scope of the disclosure.

A portion of the air management system 10, such as the air distribution system 26 for example, may be used to distribute a cleaning agent throughout the one or more areas of the aircraft 11. In an embodiment, the air distribution system 26 is capable of delivering a cleaning agent to a passenger area of the aircraft 11, such as the cabin 12 including the one or more lavatories and the galleys therein, as well as to the cockpit for example. Accordingly, use of the air distribution system 26 will clean or sterilize not only the passenger occupied areas of the aircraft 11, but also the ducts 28 of the air distribution system 26 connected thereto. However, it should be understood that any portion of the air management system 10 may be used to deliver a cleaning agent to any suitable portion of the aircraft 11.

The cleaning agent distributed throughout the aircraft may be capable of killing bacteria and/or viruses upon contact or exposure thereto. Examples of cleaning agents include but are not limited to ozone, hydrogen peroxide vapor, or another suitable aerosolized cleaning agent or disinfectant. To minimize exposure of the occupants of the aircraft 11 to such cleaning agents, a cleaning or sterilization operation during which the cleaning agent is distributed throughout the aircraft 11 will typically be performed when the aircraft 11 is grounded and/or unoccupied.

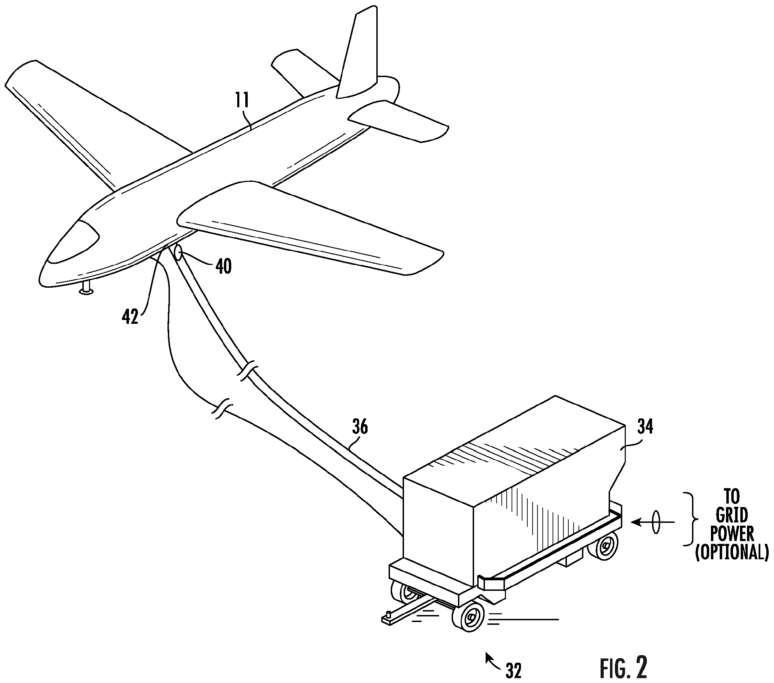

Because a cleaning operation will likely be performed when the aircraft is grounded and unoccupied, a source 40 of cleaning agent is located remotely from the aircraft 11. With reference now to FIG. 2, a modular support system 32 comprising a ground support equipment configured to couple to a parked aircraft 11 is illustrated. As shown, the support system 32 includes a cart 34 capable of supporting the aircraft by providing conditioned air, power, and the like to the aircraft 11. For instance, conditioned air may be provided from the cart 34 to the aircraft 11 along a fluid flow path defined by an airflow conduit 36 configured to couple to a portion of the air management system 10.

The cleaning source 40 is integrated into the support system 32, and specifically into the airflow conduit 36 (see FIG. 2) connectable to an inlet of the air distribution system 26. The inlet may be formed at any location along a duct 28 of the air distribution system 26, as shown in FIG. 1, or alternatively, at the air mixing unit 20, for example at a location of a ram air inlet or scoop. As shown, a cleaning source 40, such as an ozone generator operable to convert oxygen in an air flow into ozone, may be located adjacent an outlet end 42 of the airflow conduit 36 configured to couple to a portion of the aircraft 11. However, in other embodiments, a cleaning source 40 may be arranged at any position along the airflow conduit 36 extending between the cart 34 and the aircraft 11.

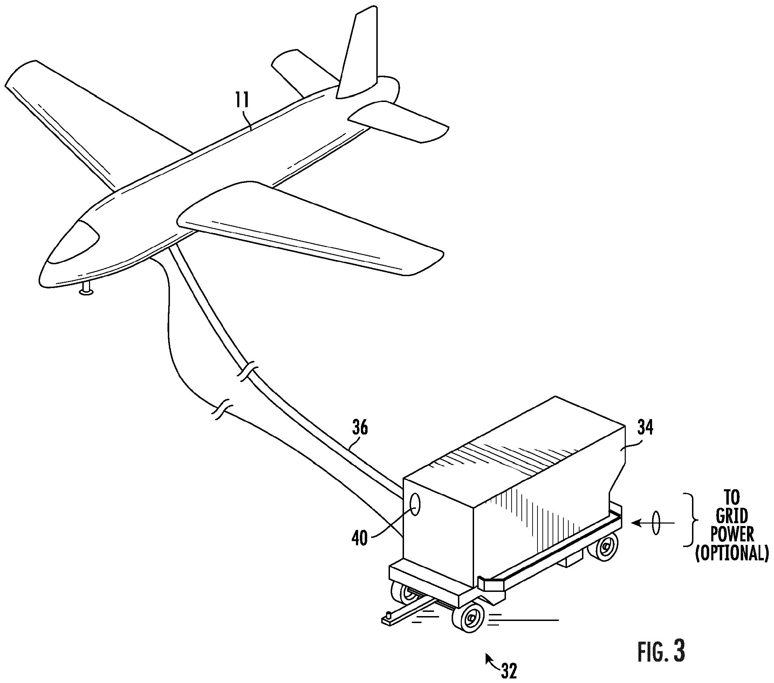

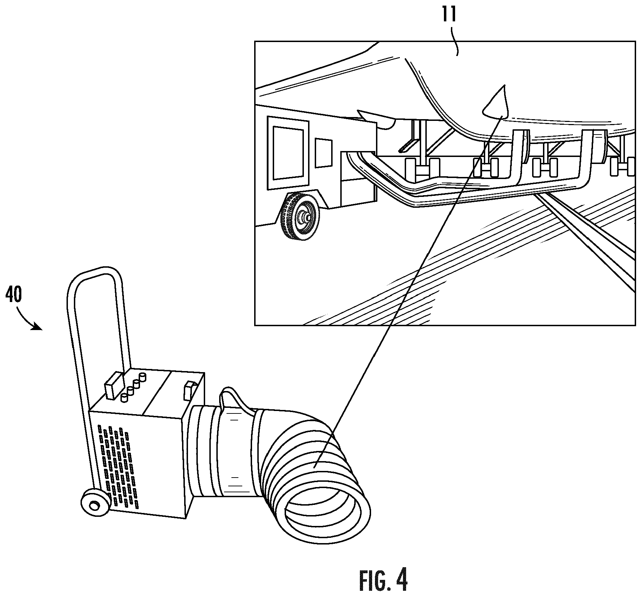

With reference now to FIG. 3, in another embodiment, the cleaning source 40 may be integrated into the cart 34 of the ground support equipment 32. The cart 34 includes an air conditioning system (not shown) for delivering conditioned air to the airflow conduit 36. However, in examples falling outside the scope of the claims, the cart 34 may deliver fresh air or non-conditioned air to the airflow conduit 36. Accordingly, in an embodiment, a cleaning source 40, such as an ozone generator for example, may be arranged at an outlet of the cart 34, such as at the interface between the cart 34 and the airflow conduit 36 for example. By positioning the cleaning source 40 at the interface between the cart 34 and the airflow conduit 36, downstream from the air conditioning system in embodiments including the air conditioning system, all of the air provided to the aircraft 11 from the cart 34 via the airflow conduit 36 is enriched with a cleaning agent via the cleaning source 40. In an example falling outside the scope of the claims, best shown in FIG. 4, in place of the ground support equipment 32, an independently operable cleaning source 40 may be directly connectable to an inlet of the air distribution system 26 of the aircraft 11.

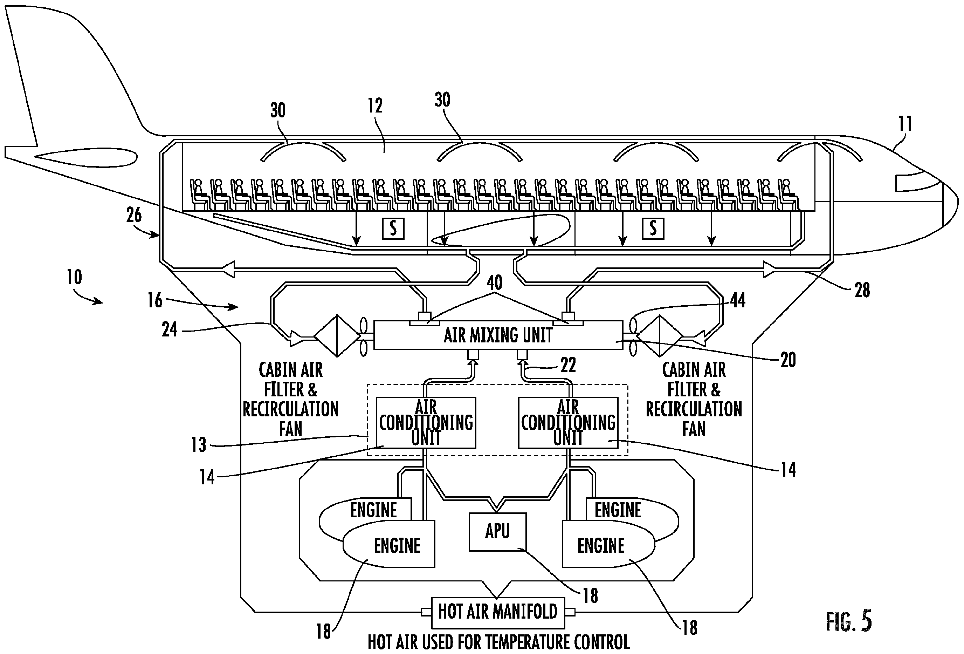

Alternatively, or in addition, a cleaning source 40 may be located on board the aircraft 11, such as integrated into a portion of the air management system 10. With reference now to FIG. 5, in an embodiment, the cleaning source 40 may be integrated into the air mixing unit 20, or an outlet thereof. As a result, any recirculation air drawn from the cabin 12, as well as any air output from the one or more air conditioning units 14 will pass through the cleaning source 40, and therefore will be enriched with cleaning agent before being delivered to the cabin 12. When the aircraft 11 is grounded, air is not typically supplied to the one or more air conditioning units 14 of the environmental control system 13, since the sources 18 are off or non-operational. Accordingly, in such embodiments, a recirculation fan 44 arranged within the one or more ducts 24 of the cabin air recirculation system 16 may be used not only to draw air from the cabin 12, but also to move air through the cleaning source 40 and back to the cabin 12.

Regardless of the location of the cleaning source 40, in operation, air having a cleaning agent entrained therein, such as ozone for example, is delivered to one or more substantially sealed areas of the aircraft 11, such as the cabin 12. As a result, the air including the cleaning agent may accumulate within and ultimately fill the entire volume of the sealed area. After a desired amount of exposure has occurred, the air will be exhausted, such as by opening one or more doors of the aircraft 11 to break the seal. The desired amount of exposure may be based on one or more parameters including, but not limited to, the concentration of the cleaning agent enriched air provided to the sealed area and the size of the cabin. In an embodiment, the desired amount of exposure may be a fixed, predetermined amount of time, selectable for example based on the model of the aircraft 11, which is indicative of the size of the cabin 12. Alternatively or in addition, one or more sensors S (Figure 5) disposed within the cabin 12 or another area being cleaned may be used to measure a concentration of the cleaning agent at the portion of the area furthest from a vent or inlet 30.

It is known that ozone decays rapidly. As a result, in embodiments where the cleaning agent is ozone, the portion of the area further from an inlet 30 will have the minimum concentration or the weakest concentration. A minimum time necessary to sterilize the area based on this sensed minimum or weakest concentration may then be used to determine when a cleaning operation is complete. In another embodiment, one or more sensors may be operable to monitor the presence of bacteria or a virus within the area, and once the sensed level falls below a threshold, the cleaning operation is complete.

As described herein, the existing infrastructure of an air management system may be used to distribute a cleaning agent through one or more desired areas of a vehicle. Such distribution and resulting cleaning is less labor intensive than a manual cleaning operation. Additionally, because the aerosolized cleaning agent is more likely to contact surfaces and areas that would otherwise have limited access with respect to manual cleaning, the cleaning process disclosed herein has improved efficacy.

The term "about" is intended to include the degree of error associated with measurement of the particular quantity based upon the equipment available at the time of filing the application.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

It is intended that the present disclosure not be limited to the particular embodiment disclosed as the best mode contemplated for carrying out this present disclosure, but that the present disclosure will include all embodiments falling within the scope of the claims.

## Claims

1. A system comprising:
a passenger area of a vehicle, wherein the vehicle is an aircraft;
an air distribution system (26) arranged in fluid communication with the passenger area;
a fluid movable through the air distribution system to the passenger area, the fluid having a cleaning agent entrained therein; **characterized by**:
a support system (32) comprising ground support equipment configured to be located remotely from the aircraft, wherein the ground support equipment is configured to connect to the aircraft when the aircraft is parked, and wherein the ground support equipment is operable to provide power and conditioned air to the aircraft; and
wherein a cleaning source (40) is integrated into the ground support equipment such that the conditioned air provided to the aircraft from the ground support equipment has the cleaning agent entrained therein.

2. The system of claim 1, wherein the cleaning source (40) contains the cleaning agent, the cleaning agent arranged in fluid communication with and upstream from the air distribution system relative to a flow of the fluid.

3. The system of claim 2, wherein the cleaning source (40) is located on board the vehicle.

4. The system of claim 3, wherein the vehicle includes an air mixing unit (20) and an outlet of the air mixing unit is connected to the air distribution system, the cleaning source being located within the air mixing unit.

5. The system of claim 2, wherein the cleaning source (40) is located outside the vehicle.

6. The system of claim 5, wherein the support system (32) connectable to the air distribution system, the support system including:
a cart (34) including an air conditioner; and
an airflow conduit (36) defining a fluid flow path extending from the cart to the air distribution system, wherein the cleaning source is integrated into the support system.

7. The system of claim 6, wherein the cleaning source (40) is disposed along the fluid flow path; or wherein the cleaning source (40) is integrated into the cart downstream from the air conditioner relative to a conditioned fluid flow.

8. The system of any preceding claim, wherein the passenger area is a cabin of the aircraft.

9. The system of any preceding claim, wherein the cleaning agent is ozone; or wherein the cleaning agent is hydrogen peroxide vapor.

10. A cleaning system for sterilizing a passenger area of a vehicle, the cleaning system comprising the system of claim 1 and the cleaning system further comprising:
the cleaning source (40) operable to generate an aerosolized cleaning agent entrained within an air flow, the cleaning source being connectable to the air distribution system of the vehicle.

11. The cleaning system of claim 10, wherein the support system (32) connectable to the air distribution system, the support system including:
a cart (34) including an air conditioner; and
an airflow conduit (36) defining a fluid flow path extending from the cart to the air distribution system, wherein the cleaning source being integrated into the support system.

12. The cleaning system of claim 11, wherein the cleaning source (40) is disposed along the fluid flow path; or wherein the cleaning source (40) is integrated into the cart downstream from the air conditioner relative to a conditioned fluid flow.

13. A method of sterilizing a passenger area of a vehicle, wherein the vehicle is an aircraft, the method comprising:
providing a ground support equipment located remotely from the aircraft, wherein the ground support equipment is operable to provide power and conditioned air to the aircraft;
fluidly connecting a cleaning source to an air distribution system of the aircraft, wherein the cleaning source is integrated into the ground support equipment such that the conditioned air provided to the aircraft from the ground support equipment has a cleaning agent entrained therein;
sealing the passenger area;
operating the cleaning source to provide a fluid including the cleaning agent to the passenger area; and
exposing the passenger area to the fluid including aerosolized cleaning agent for a predetermined length of time to kill bacteria within the passenger area.

14. The method of claim 13, wherein the predetermined length of time is calculated based on a size of the passenger area and a concentration of the aerosolized cleaning agent within the passenger area.

15. The method of claim 13, wherein the predetermined length of time is determined in response to a sensed concentration of the aerosolized cleaning agent within the passenger area, and preferably wherein the cleaning source is operated until a sensed level of bacteria falls below a threshold.

## Patentansprüche

1. System, umfassend:
einen Passagierbereich eines Fahrzeugs, wobei das Fahrzeug ein Luftfahrzeug ist;
ein Luftverteilungssystem (26), das in Fluidverbindung mit dem Passagierbereich angeordnet ist;
ein Fluid, das durch das Luftverteilungssystem hindurch zu dem Passagierbereich bewegbar ist, wobei das Fluid ein darin mitgeführtes Reinigungsmittel aufweist; **gekennzeichnet durch**:
ein Unterstützungssystem (32), welches ein Bodenunterstützungsgerät umfasst, das dazu konfiguriert ist, sich entfernt von dem Luftfahrzeug zu befinden, wobei das Bodenunterstützungsgerät dazu konfiguriert ist, sich mit dem Luftfahrzeug zu verbinden, wenn das Luftfahrzeug geparkt ist, und wobei das Bodenunterstützungsgerät betreibbar ist, um dem Luftfahrzeug Strom und klimatisierte Luft bereitzustellen; und
wobei eine Reinigungsquelle (40) in das Bodenunterstützungsgerät integriert ist, damit die klimatisierte Luft, die dem Luftfahrzeug von dem Bodenunterstützungsgerät bereitgestellt wird, das Reinigungsmittel darin mitführt.

2. System nach Anspruch 1, wobei die Reinigungsquelle (40) das Reinigungsmittel enthält, wobei das Reinigungsmittel in Fluidverbindung mit und stromaufwärts von dem Luftverteilungssystem relativ zu einem Strom des Fluids angeordnet ist.

3. System nach Anspruch 2, wobei sich die Reinigungsquelle (40) an Bord des Fahrzeugs befindet.

4. System nach Anspruch 3, wobei das Fahrzeug eine Luftmischeinheit (20) beinhaltet und ein Auslass der Luftmischeinheit mit dem Luftverteilungssystem verbunden ist, wobei sich die Reinigungsquelle innerhalb der Luftmischeinheit befindet.

5. System nach Anspruch 2, wobei sich die Reinigungsquelle (40) außerhalb des Fahrzeugs befindet.

6. System nach Anspruch 5, wobei das Unterstützungssystem (32) mit dem Luftverteilungssystem verbindbar ist, wobei das Unterstützungssystem Folgendes beinhaltet:
einen Wagen (34), der eine Klimaanlage beinhaltet; und
eine Luftstromleitung (36), die einen Fluidstromweg definiert, der sich von dem Wagen zu dem Luftverteilungssystem erstreckt, wobei die Reinigungsquelle in das Unterstützungssystem integriert ist.

7. System nach Anspruch 6, wobei die Reinigungsquelle (40) entlang des Fluidstromwegs angeordnet ist; oder wobei die Reinigungsquelle (40) relativ zu einem klimatisierten Fluidstrom stromabwärts von der Klimaanlage in den Wagen integriert ist.

8. System nach einem der vorhergehenden Ansprüche, wobei der Passagierbereich eine Kabine des Luftfahrzeugs ist.

9. System nach einem der vorhergehenden Ansprüche, wobei das Reinigungsmittel Ozon ist; oder wobei das Reinigungsmittel Wasserstoffperoxiddampf ist.

10. Reinigungssystem zum Sterilisieren eines Passagierbereichs eines Fahrzeugs, wobei das Reinigungssystem das System nach Anspruch 1 umfasst und das Reinigungssystem ferner Folgendes umfasst:
die Reinigungsquelle (40), die betreibbar ist, um ein innerhalb eines Luftstroms mitgeführtes aerosoliertes Reinigungsmittel zu erzeugen, wobei die Reinigungsquelle mit dem Luftverteilungssystem des Fahrzeugs verbindbar ist.

11. Reinigungssystem nach Anspruch 10, wobei das Unterstützungssystem (32) mit dem Luftverteilungssystem verbindbar ist, wobei das Unterstützungssystem Folgendes beinhaltet:
einen Wagen (34), der eine Klimaanlage beinhaltet; und
eine Luftstromleitung (36), die einen Fluidstromweg definiert, der sich von dem Wagen zu dem Luftverteilungssystem erstreckt, wobei die Reinigungsquelle in das Unterstützungssystem integriert ist.

12. Reinigungssystem nach Anspruch 11, wobei die Reinigungsquelle (40) entlang des Fluidstromwegs angeordnet ist; oder wobei die Reinigungsquelle (40) relativ zu einem klimatisierten Fluidstrom stromabwärts von der Klimaanlage in den Wagen integriert ist.

13. Verfahren zum Sterilisieren eines Passagierbereichs eines Fahrzeugs, wobei das Fahrzeug ein Luftfahrzeug ist, wobei das Verfahren Folgendes umfasst:
Bereitstellen eines Bodenunterstützungsgeräts, das entfernt von dem Luftfahrzeug angeordnet ist, wobei das Bodenunterstützungsgerät betreibbar ist, um dem Luftfahrzeug Strom und klimatisierte Luft bereitzustellen; fluidisches Verbinden einer Reinigungsquelle mit einem Luftverteilungssystem des Luftfahrzeugs, wobei die Reinigungsquelle in das Bodenunterstützungsgerät integriert ist, damit die klimatisierte Luft, die dem Luftfahrzeug von dem Bodenunterstützungsgerät bereitgestellt wird, ein darin mitgeführtes Reinigungsmittel aufweist;
Abdichten des Passagierbereichs;
Betreiben der Reinigungsquelle, um dem Passagierbereich ein Fluid bereitzustellen, welches das Reinigungsmittel beinhaltet; und
Aussetzen des Passagierbereichs dem Fluid, welches ein aerosoliertes Reinigungsmittel beinhaltet, für einen vorbestimmten Zeitraum, um Bakterien innerhalb des Passagierbereichs abzutöten.

14. Verfahren nach Anspruch 13, wobei der vorbestimmte Zeitraum basierend auf einer Größe des Passagierbereichs und einer Konzentration des aerosolierten Reinigungsmittels innerhalb des Passagierbereichs berechnet wird.

15. Verfahren nach Anspruch 13, wobei der vorbestimmte Zeitraum als Reaktion auf eine erfasste Konzentration des aerosolierten Reinigungsmittels innerhalb des Passagierbereichs bestimmt wird und wobei vorzugsweise die Reinigungsquelle betrieben wird, bis ein gemessener Bakteriengehalt unter einen Schwellenwert fällt.

## Revendications

1. Système comprenant :
une zone passagers d'un véhicule, dans lequel le véhicule est un aéronef ;
un système de distribution d'air (26) agencé en communication fluidique avec la zone passagers ;
un fluide mobile à travers le système de distribution d'air jusqu'à la zone passagers, le fluide ayant un agent de nettoyage entraîné dans celui-ci ; **caractérisé par** :
un système d'assistance (32) comprenant un équipement d'assistance au sol configuré pour être situé à distance de l'aéronef, dans lequel l'équipement d'assistance au sol est configuré pour se connecter à l'aéronef lorsque l'aéronef est stationné, et dans lequel l'équipement d'assistance au sol peut fonctionner pour fournir de l'énergie et de l'air conditionné à l'aéronef ; et
dans lequel une source de nettoyage (40) est intégrée dans l'équipement d'assistance au sol de sorte que l'air conditionné fourni à l'aéronef depuis l'équipement d'assistance au sol ait l'agent de nettoyage entraîné dans celui-ci.

2. Système selon la revendication 1, dans lequel la source de nettoyage (40) contient l'agent de nettoyage, l'agent de nettoyage étant disposé en communication fluidique avec et en amont du système de distribution d'air par rapport à un écoulement du fluide.

3. Système selon la revendication 2, dans lequel la source de nettoyage (40) est située à bord du véhicule.

4. Système selon la revendication 3, dans lequel le véhicule comporte une unité de mélange d'air (20) et une sortie de l'unité de mélange d'air est connectée au système de distribution d'air, la source de nettoyage étant située à l'intérieur de l'unité de mélange d'air.

5. Système selon la revendication 2, dans lequel la source de nettoyage (40) est située à l'extérieur du véhicule.

6. Système selon la revendication 5, dans lequel le système d'assistance (32) peut être connecté au système de distribution d'air, le système d'assistance comportant :
un chariot (34) comportant un climatiseur ; et
un conduit d'écoulement d'air (36) définissant un trajet d'écoulement de fluide s'étendant du chariot au système de distribution d'air, dans lequel la source de nettoyage est intégrée dans le système d'assistance.

7. Système selon la revendication 6, dans lequel la source de nettoyage (40) est disposée le long du trajet d'écoulement de fluide ; ou dans lequel la source de nettoyage (40) est intégrée dans le chariot en aval du climatiseur par rapport à un écoulement de fluide conditionné.

8. Système selon une quelconque revendication précédente, dans lequel la zone passagers est une cabine de l'aéronef.

9. Système selon une quelconque revendication précédente, dans lequel l'agent de nettoyage est l'ozone ; ou dans lequel l'agent de nettoyage est la vapeur de peroxyde d'hydrogène.

10. Système de nettoyage pour stériliser une zone passagers d'un véhicule, le système de nettoyage comprenant le système selon la revendication 1 et le système de nettoyage comprenant en outre :
la source de nettoyage (40) pouvant fonctionner pour générer un agent de nettoyage en aérosol entraîné dans un flux d'air, la source de nettoyage pouvant être connectée au système de distribution d'air du véhicule.

11. Système selon la revendication 10, dans lequel le système d'assistance (32) peut être connecté au système de distribution d'air, le système d'assistance comportant :
un chariot (34) comportant un climatiseur ; et
un conduit d'écoulement d'air (36) définissant un trajet d'écoulement de fluide s'étendant du chariot au système de distribution d'air, dans lequel la source de nettoyage est intégrée dans le système d'assistance.

12. Système de nettoyage selon la revendication 11, dans lequel la source de nettoyage (40) est disposée le long du trajet d'écoulement de fluide ; ou dans lequel la source de nettoyage (40) est intégrée dans le chariot en aval du climatiseur par rapport à un écoulement de fluide conditionné.

13. Procédé de stérilisation d'une zone passagers d'un véhicule, dans lequel le véhicule est un aéronef, le procédé comprenant :
la fourniture d'un équipement d'assistance au sol situé à distance de l'aéronef, dans lequel l'équipement d'assistance au sol peut fonctionner pour fournir de l'énergie et de l'air conditionné à l'aéronef ; la connexion fluidique d'une source de nettoyage à un système de distribution d'air de l'aéronef, dans lequel la source de nettoyage est intégrée dans l'équipement d'assistance au sol de sorte que l'air conditionné fourni à l'aéronef depuis l'équipement d'assistance au sol contient un agent de nettoyage entraîné dans celui-ci ;
le scellage de la zone passagers ;
le fait de faire fonctionner la source de nettoyage pour fournir un fluide comportant l'agent de nettoyage à la zone passagers ; et
l'exposition de la zone passagers au fluide comportant un agent de nettoyage en aérosol pendant une durée prédéterminée pour tuer les bactéries dans la zone passagers.

14. Procédé selon la revendication 13, dans lequel la durée prédéterminée est calculée sur la base d'une taille de la zone passagers et d'une concentration de l'agent de nettoyage en aérosol dans la zone passagers.

15. Procédé selon la revendication 13, dans lequel la durée prédéterminée est déterminée en réponse à une concentration détectée de l'agent de nettoyage en aérosol dans la zone passagers, et de préférence dans lequel la source de nettoyage est utilisée jusqu'à ce qu'un niveau détecté de bactéries tombe en dessous d'un seuil.
